# EUROPEAN PATENT APPLICATION

(11) **EP 3 225 149 A1**
(43) Date of publication of application: **04.10.2017**
(21) Application number: 16841181.7
(22) Date of filing: 12.04.2016
(51) Int. Cl.: A61B 1/00

(54) **OBSERVATION OPTICAL SYSTEM UNIT, IMAGING UNIT, AND ENDOSCOPE**

(30) Priority: 01.09.2015 JP 2015172202
(71) Applicant: Olympus Corporation, Hachioji-shi Tokyo 192-8507 (JP)
(72) Inventor: WATAYA, Yuichi, Hachioji-shi Tokyo 192-8507 (JP)
(74) Representative: Schicker, Silvia
(86) International application number: PCT/JP2016/061765
(87) International publication number: WO 2017/038149

(57) **Abstract**

An observation optical system unit includes a first lens 58a which is positioned closest to an object side, among rear-group lenses 58, and which is configured by a stepped lens including a proximal portion 58b, an outer circumference of which is held by a rear-group lens frame 57, and a protruding portion 58c formed to have an outer circumference smaller than the outer circumference of the proximal portion 58b and protruding into a region At where a moving lens frame 65 is allowed to advance and retract, where the moving lens frame 65 overlaps with the protruding portion 58c at a retracted position.

## Description

### Technical Field

The present invention relates to an observation optical system unit which allows an optical property of an observation optical system to be changed, an image pickup unit, and an endoscope.

### Background Art

As is well known, endoscopes are widely used for observation, treatments or the like of inside of a living body (inside a body cavity), or for inspection, repair and the like in an industrial plant facility.

In recent years, an image pickup unit used for such an endoscope sometimes includes an observation optical system unit which is capable of changing the focal length by moving an observation optical system in the direction of a photographing optical axis so as to realize focus adjustment for a photography image or a zooming function for performing magnification adjustment regarding wide/tele, for example. Note that the technique for such an observation optical system unit allowing the focal length to be changed is used in various photographing devices without being limited to endoscopes.

Particularly, an observation optical system unit of a focus switching type which is focused when a moving lens unit (moving lens frame) is at a predetermined advanced position or retracted position is widely used for endoscopes requiring miniaturization. When assembling an image pickup unit provided with such an observation optical system unit of a focus switching type, the advanced position and the retracted position of the moving lens frame (that is, the relative position of the moving lens frame with respect to each fixed lens frame or the like in the direction of the photographing optical axis) have to be finely adjusted so as to achieve an appropriately focused state even when there is a machining tolerance of each lens.

On the other hand, for example, Japanese Patent Application Laid-Open Publication No. 2003-310531 discloses a technique of performing focusing adjustment by mounting, on an outer circumferential portion of a rear-group lens frame, a ring member having an inclined portion formed on a distal end side, rotating the ring member in the circumferential direction and changing the adjustment position of a moving lens frame at the time of TELE (narrow angle), and then, fixing the ring member by an adhesive.

Image pickup units of recent years tend to have resolution significantly reduced at a part of an image due to shrinking of the pixel pitch of an image pickup device, even if focusing adjustment as described above is performed. Such reduction in the resolution is caused mainly by tilting of a moving lens frame with respect to the photographing optical axis, and becomes a problem for an endoscope especially at the time of observation of a microvessel on the tele side, for example.

Accordingly, the moving lens frame may be set to be long in the direction of the photographing optical axis with the aim of preventing tilting of the moving lens frame. However, if the moving lens frame is set to be long, the length of the entire image pickup unit is increased. Accordingly, for example, if such an image pickup unit is applied to an endoscope, the size of a rigid distal end portion is increased, possibly leading to reduction in the operability for the user or the like.

The present invention has been made in view of the above circumstances, and has its object to provide an observation optical system unit which is capable of picking up an image without reducing the resolution by preventing tilting of a moving lens frame while not causing an increase in the size, an image pickup unit, and an endoscope.

### Disclosure of Invention

### Means for Solving the Problem

An observation optical system unit according to an aspect of the present invention includes an observation optical system, of a focus switching type, including front-group lenses, a moving lens, and rear-group lenses in order from an object side, a fixed frame holding the front-group lenses and the rear-group lenses, and a moving frame holding the moving lens, the moving frame slidingly contacting an inside of the fixed frame, and being capable of advancing and retracting along an optical axis direction of the observation optical system, wherein a first lens positioned closest to the object side, among the rear-group lenses, is configured by a stepped lens including a proximal portion, an outer circumference of which is held by the fixed frame, and a protruding portion formed to have an outer circumference smaller than the outer circumference of the proximal portion and protruding into a region where the moving frame is allowed to advance and retract, and the moving frame overlaps with the protruding portion at least at a position retracted to a side of the rear-group lenses along an optical axis of the observation optical system.

An image pickup unit according to an aspect of the present invention includes the observation optical system unit, and an image pickup device in which a subject image is formed by the observation optical system unit.

An endoscope according to an aspect of the present invention includes the image pickup unit at a distal end portion of an insertion section inserted into a subject.

### Brief Description of the Drawings

Fig. 1 is an explanatory diagram showing an overall configuration of an endoscope;
Fig. 2 is a cross-sectional view showing internal configurations of a distal end portion and a bending portion;
Fig. 3 is a cross-sectional view showing a configuration of an image pickup unit in a state where a moving lens unit is moved to an advanced position at the front;
Fig. 4 is a cross-sectional view showing the configuration of the image pickup unit in a state where the moving lens unit is moved to a retracted position at the rear;
Fig. 5 is an enlarged cross-sectional view showing main parts of rear-group lenses held by a rear-group lens frame;
Fig. 6 is a perspective view showing a relationship between a lens at a most distal end, among the rear-group lenses, and a moving lens frame;
Fig. 7 is an enlarged cross-sectional view showing a modification of the lens at the most distal end, among the rear-group lenses;
Fig. 8 is a perspective view showing a modification of the relationship between the lens at the most distal end, among the rear-group lenses, and the moving lens frame;
Fig. 9 is a cross-sectional view showing a modification of the configuration of the image pickup unit in a state where the moving lens unit is moved to the advanced position at the front; and
Fig. 10 is a cross-sectional view showing a modification of the configuration of the image pickup unit in a state where the moving lens unit is moved to the retracted position at the rear.

### Best Mode for Carrying Out the Invention

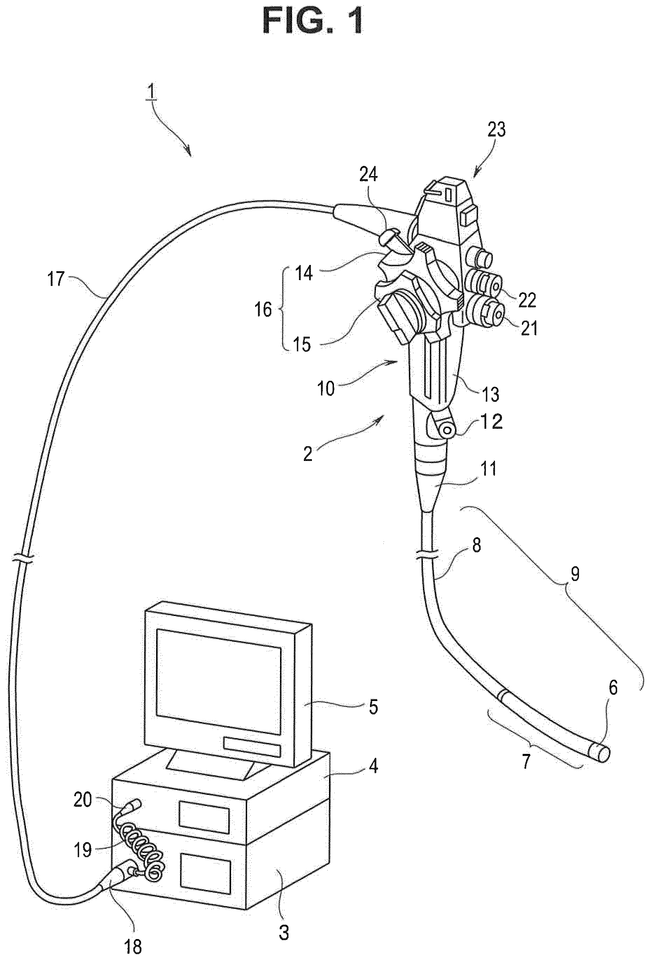

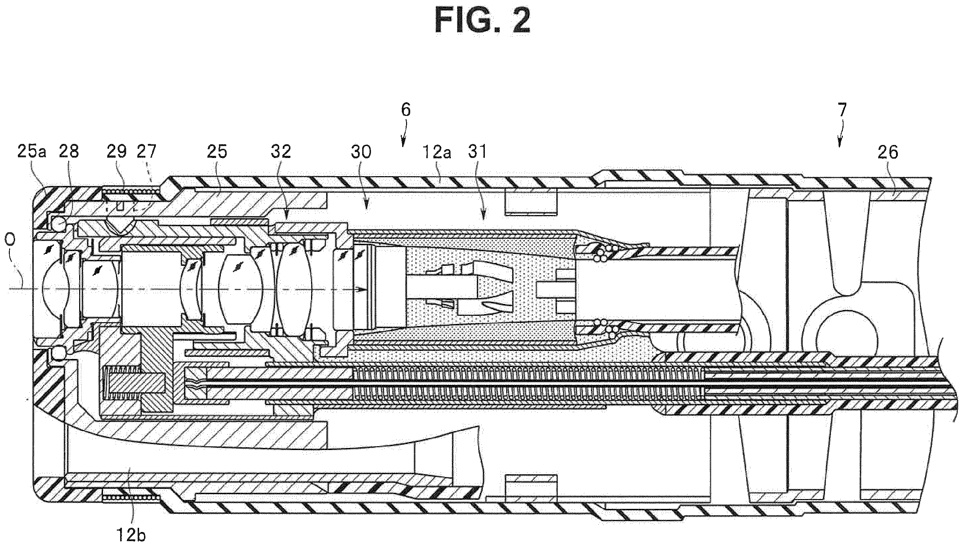

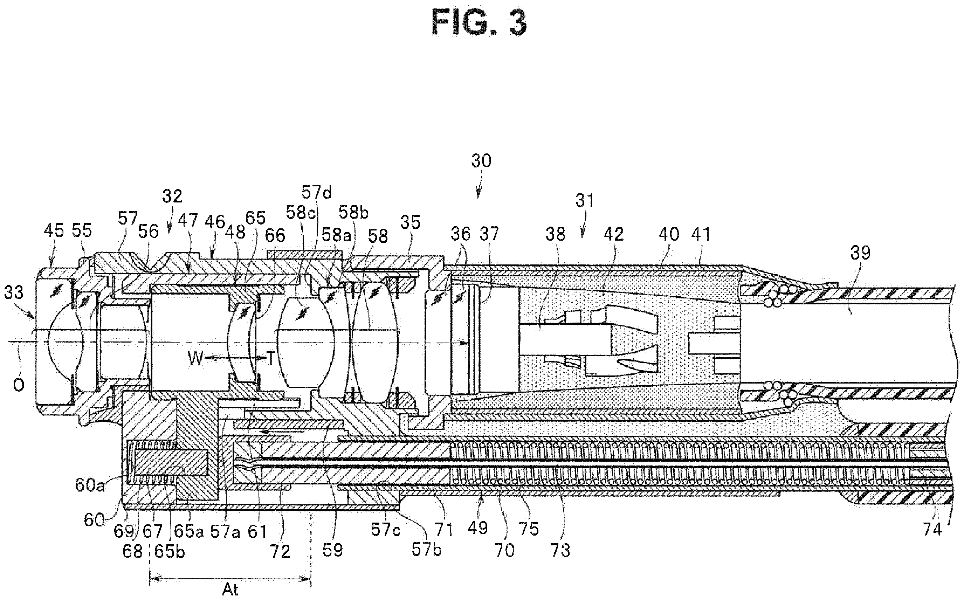

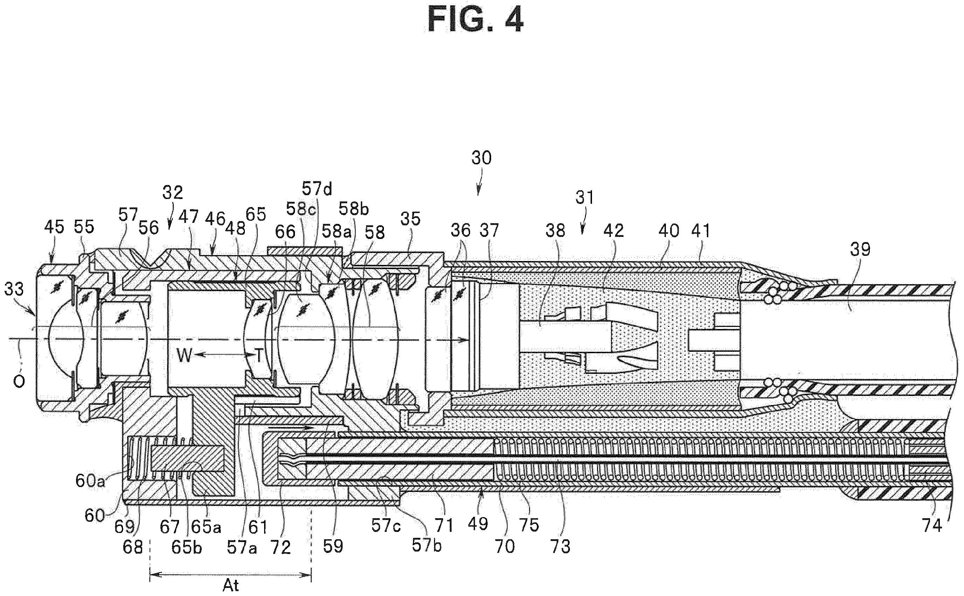

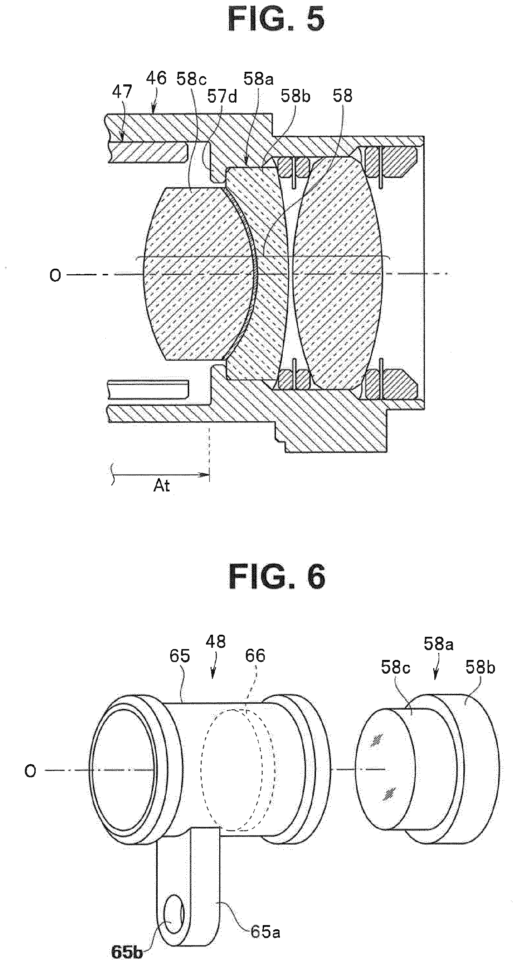

Hereinafter, a mode of the present invention will be described with reference to the drawings. The drawings relate to an embodiment of the present invention, and Fig. 1 is an explanatory diagram showing an overall configuration of an endoscope, Fig. 2 is a cross-sectional view showing internal configurations of a distal end portion and a bending portion, Fig. 3 is a cross-sectional view showing a configuration of an image pickup unit in a state where a moving lens unit is moved to an advanced position at the front, Fig. 4 is a cross-sectional view showing the configuration of the image pickup unit in a state where the moving lens unit is moved to a retracted position at the rear, Fig. 5 is an enlarged cross-sectional view showing main parts of rear-group lenses held by a rear-group lens frame, and Fig. 6 is a perspective view showing a relationship between a lens at a most distal end, among the rear-group lenses, and a moving lens frame.

As shown in Fig. 1, an electronic endoscope system (hereinafter simply referred to as an endoscope system) 1 of the present embodiment includes an electronic endoscope apparatus (hereinafter simply referred to as an endoscope) 2 as an endoscope, a light source device 3, a video processor 4, and a color monitor 5 which are electrically connected to one another.

The endoscope 2 includes an insertion section 9, and an operation section 10 from which the insertion section 9 extends, and a universal cord 17 extending from the operation section 10 is connected to the light source device 3 via a scope connector 18. Also, a coil-shaped scope cable 19 extends from the scope connector 18. Moreover, an electrical connector section 20 is provided on the other end side of the scope cable 19, and the electrical connector section 20 is connected to the video processor 4.

The insertion section 9 includes a distal end portion 6, a bending portion 7, and a flexible tube portion 8 which are provided in order in a continuous manner from the distal end side. At a distal end surface of the distal end portion 6, a distal end opening portion, an observation window, a plurality of illumination windows, a joint window cleaning port, and an observation object cleaning port, which are well-known, are disposed (none of which is shown in the drawing).

Inside the distal end portion 6, an image pickup unit described below is disposed on a rear surface side of the observation window. Furthermore, a distal end side of a light guide bundle, not shown, is disposed on a rear surface side of the plurality of illumination windows.

The light guide bundle is inserted and disposed inside the universal cord 17 from the insertion section 9 via the operation section 10, and when the scope connector 18 is connected to the light source device 3, the light guide bundle is capable of transmitting illumination light from the light source device 3 to the illumination windows.

The observation window cleaning port and the observation object cleaning port form opening portions of two cleaning tubes, not shown, which are inserted inside the universal cord 17 from the distal end portion 6.

The cleaning tubes are connected, on the light source device 3 side, to a cleaning tank containing cleaning water and a compressor (neither is shown in the drawing).

The operation section 10 includes a bend preventing portion 11 from which the insertion section 9 extends, a forceps port 12 disposed on a side portion on a lower portion side, an operation section main body 13 forming a grip portion at a mid-portion, a bending operation section 16 including two bending operation knobs 14, 15 provided on an upper portion side, an air/water feeding control section 21, a suction control section 22, a switch section 23 including a plurality of switches to mainly operate an image pickup function, and an operation lever 24 configured to operate a focusing function for focus adjustment or a zooming function for performing magnification adjustment regarding wide/tele by advancing or retracting a moving lens provided in the image pickup unit described below, for example.

Note that the forceps port 12 of the operation section 10 configures an opening portion of a treatment instrument channel, not shown, inserted and disposed mainly in the insertion section 9 up to a distal end opening portion of the distal end portion 6.

Next, the configuration of the distal end portion 6 of the endoscope 2 will be mainly described with reference to Fig. 2.

As shown in Fig. 2, an image pickup unit 30 is disposed inside the distal end portion 6.

The image pickup unit 30 is fitted and disposed in a distal end rigid member 25 having rigidity, and is fixed to the distal end rigid member 25 from a side surface direction by a set screw 27.

Furthermore, an O-shaped ring 28 configured to maintain water-tightness to the distal end rigid member 25 is disposed on an outer circumferential portion on a distal end side of the image pickup unit 30. A distal end cover 25a configuring a distal end surface of the distal end portion 6 is bonded and fixed on a distal end side of the distal end rigid member 25.

Note that, as described above, the distal end opening portion, which is a hole portion formed in the distal end cover 25a, configures an opening portion of a treatment instrument channel 12b inside the distal end portion 6.

Furthermore, a plurality of bending pieces 26 configuring the bending portion 7 are provided continuously to a proximal end side of the distal end rigid member 25, and outer circumferences of the distal end rigid member 25 and the bending pieces 26 are integrally covered by a distal end insertion section rubber member 12a. A distal end outer circumferential portion of the distal end insertion section rubber member 12a is fixed to the distal end rigid member 25 by a bobbin bonding section 29.

Note that members such as the cleaning tubes and the light guide bundle for illumination which are disposed in the distal end portion 6 are well-known components, and description of such components is omitted.

Next, a detailed configuration of the image pickup unit 30 will be described with reference to Figs. 3 to 5.

As shown in Figs. 3 and 4, the image pickup unit 30 of the present embodiment is configured by including a solid-state image pickup device unit 31, and an observation optical system unit 32 which is provided continuously to a distal end side of the solid-state image pickup device unit 31.

The solid-state image pickup device unit 31 includes a solid-state image pickup device holding frame 35, and a front surface side of a solid-state image pickup device chip 37 such as a CCD or a CMOS is held by the solid-state image pickup device holding frame 35 via an optical member 36 such as a cover glass.

Furthermore, a laminated substrate 38 is electrically connected to a rear surface side of the solid-state image pickup device chip 37 via an FPC, not shown.

Moreover, a plurality of communication lines branched from a cable 39 are connected to the laminated substrate 38. The cable 39 is inserted and disposed inside the endoscope 2, and is electrically connected to the video processor via the electrical connector section 20.

Moreover, a reinforcing frame 40 is provided in a continuous manner on an outer circumferential portion on a proximal end side of the solid-state image pickup device holding frame 35, and a heat-shrinkable tube 41 that covers up to a distal end portion of the cable 39 is provided on an outer circumference of the reinforcing frame 40.

Note that a protection agent 42, such as an adhesive, for maintaining water-tightness of the solid-state image pickup device unit 31 and protecting the solid-state image pickup device unit 31 fills a space formed by the reinforcing frame 40 and the heat-shrinkable tube 41 from a proximal end part of the solid-state image pickup device holding frame 35.

The observation optical system unit 32 of the present embodiment is configured by including an observation optical system 33 of a two-focus type which realizes the focusing function or the zooming function by advancing or retracting a lens provided inside and changing the optical property (focal length).

More specifically, the observation optical system unit 32 is configured by including a front-group lens unit 45 positioned on a distal end side, a rear-group lens unit 46 positioned in a continuous manner on a proximal end side of the front-group lens unit 45, a guide frame 47 interposed between the front-group lens unit 45 and the rear-group lens unit 46, a moving lens unit 48 which is capable of advancing or retracting inside the guide frame 47 along the direction of a photographing optical axis O, and an actuator 49 which advances or retracts the moving lens unit 48.

The front-group lens unit 45 is configured by including a front-group lens frame 55 which is a fixed frame, and front-group lenses 56 including a plurality of fixed lenses held by the front-group lens frame 55.

The rear-group lens unit 46 is configured by including a rear-group lens frame 57 which is a fixed frame, a distal end side of which is inserted over the front-group lens frame 55, and rear-group lenses 58 including a plurality of fixed lenses held on a proximal end side of the rear-group lens frame 57 and on the photographing optical axis O.

The solid-state image pickup device holding frame 35 is inserted over the proximal end side of the rear-group lens frame 57, and the solid-state image pickup device unit 31 and the observation optical system unit 32 are thereby coupled to each other.

Furthermore, the rear-group lens frame 57 has a slit 57a penetrating from an inner circumferential side of the rear-group lens to an outer circumferential side frame 57. The slit 57a extends in the same direction as the direction of the photographing optical axis O, and a distal end side of the slit 57a is open at a distal end of the rear-group lens frame 57.

Moreover, a holding rod 57b protruding in an outer diameter direction on a proximal end side of the slit 57a is provided to the rear-group lens frame 57, and an actuator holding hole 57c penetrating in the same direction as the photographing optical axis O is provided to the holding rod 57b.

Moreover, an adjustment ring 59 which enables accurate positioning of the moving lens unit 48 at a stop position on the proximal end side in the direction of the photographing optical axis O (i.e., a retracted position) is provided to the rear-group lens frame 57.

The adjustment ring 59 is configured by a C-shaped ring member, a width of which in the direction of the photographing optical axis O linearly changes along a circumferential direction, and is disposed with a proximal end abutted against the holding rod 57b. The adjustment ring 59 allows the relative positions of the moving lens unit 48 and the holding rod 57b (that is, the retracted position of the moving lens unit 48) to be changed by being rotated around the photographing optical axis O along an outer circumferential surface of the rear-group lens frame 57.

For example, as shown in Figs. 3 to 6, a first lens 58a positioned closest to an object side (i.e., at a most distal end side), among a plurality of lenses configuring the rear-group lenses 58, is configured by including a proximal portion 58b, an outer circumference of which is held by the rear-group lens frame 57, and a protruding portion 58c protruding on a distal end side from the proximal portion 58b. An outer circumference of the protruding portion 58c of the first lens 58a is set to be smaller than the outer circumference of the proximal portion 58b, and the protruding portion 58c protrudes into a region At where the moving lens unit 48 can advance or retract.

That is, the observation optical system 33 of the present embodiment is designed in such a way that an effective diameter ϕ1 of an incident surface of the first lens 58a is smaller than an effective diameter ϕ2 of an emission surface by appropriate specification setting of optical members such as various lenses. Such a difference in the effective diameters is used to configure the first lens 58a by a stepped lens having no unnecessary part, on the incident side, which does not optically function. More specifically, the first lens 58a of the present embodiment is configured by bonding together, by a transparent adhesive or the like, the protruding portion 58c, which is a convex lens with a small diameter, and the proximal portion 58b, which is a concave lens with a large diameter.

To perform positioning of the first lens 58a, the rear-group lens frame 57 is provided with an abutting portion 57d having an inward flange shape which protrudes in an inner diameter direction. The first lens 58a is positioned with respect to the rear-group lens frame 57, in the direction of the optical axis O, by a step portion formed by the proximal portion 58b and the protruding portion 58c abutting against a surface on a proximal end side of the abutting portion 57d in the direction of the optical axis O.

The guide frame 47 is interposed between an outer circumferential side of the front-group lens frame 55 and an inner circumferential side of the rear-group lens frame 57. A front-side stopper 60 protruding in an outer diameter direction is provided on the distal end side of the guide frame 47. The front-side stopper 60 faces the holding rod 57b on the distal end side of the rear-group lens frame 57, and a spring receiving portion 60a which faces the actuator holding hole 57c of the holding rod 57b is provided to the front-side stopper 60 in a recessed manner. Also, the guide frame 47 has a slit 61 penetrating from an inner circumferential side of the guide frame 47 to an outer circumferential side of the guide frame 47. The slit 61 extends in the same direction as the direction of the photographing optical axis O in a manner overlapping with the slit 57a of the rear-group lens frame 57, and a proximal end side of the slit 61 is open at a proximal end of the guide frame 47.

A proximal end side of the guide frame 47 extends to near the abutting portion 57d so as to prevent tilting with respect to the fixed frame (that is, the front-group lens frame 55 and the rear-group lens frame 57) while securing a sufficient length in the direction of the photographing optical axis O. Moreover, such an extended proximal end portion of the guide frame 47 overlaps with the protruding portion 58c of the first lens 58a while being at a position separated by a predetermined gap in the outer diameter direction.

The moving lens unit 48 is configured by including a moving lens frame 65, which is a moving frame, and a moving lens 66 which is held by the moving lens frame 65.

In the present embodiment, the moving lens frame 65 slidingly contacts an inner circumferential surface of the guide frame 47, and is guided along the direction of the photographing optical axis O. That is, the moving lens frame 65 slidingly contacts the inside of the rear-group lens frame 57, which is a fixed frame, across the guide frame 47, and is enabled to advance or retract in the direction along the photographing optical axis O.

To secure a sufficient length in the direction of the photographing optical axis O, and prevent tilting with respect to the fixed frame (that is, the front-group lens frame 55 and the rear-group lens frame 57), a distal end side and a proximal end side of the moving lens frame 65 extend further than the moving lens 66 in the direction of the photographing optical axis O. An inner diameter of the proximal end side of the moving lens frame 65 is formed larger than an outer diameter of the protruding portion 58c of the first lens 58a, and may overlap with the protruding portion 58c of the first lens 58a on an inner circumferential side of the guide frame 47 when the moving lens frame 65 is positioned at the retracted position, as shown in Fig. 4.

An operation rod 65a protruding in the outer circumferential direction is provided to the moving lens frame 65. The operation rod 65a protrudes to an outer circumferential side of the rear-group lens frame 57 through the slit 57a of the rear-group lens frame 57 and the slit 61 of the guide frame 47, and faces the holding rod 57b and the front-side stopper 60. A bearing portion 65b facing the spring receiving portion 60a is provided on a side of the operation rod 65a facing the front-side stopper 60, and a shaft member 67 protruding into the spring receiving portion 60a is held in the bearing portion 65b. Moreover, a return spring 68, one end side of which is held inside the spring receiving portion 60a, is wound around an outer circumference of the shaft member 67, and the operation rod 65a is biased toward a proximal end side (holding rod 57b side) by the return spring 68.

Note that a cover 69 configured to block the slits 57a, 61 in a water-tight manner is provided at a protruding end side of the holding rod 57b, the front-side stopper 60 and the operation rod 65a.

The actuator 49 includes a guide tube 70, a distal end side of which is held in the actuator holding hole 57c of the holding rod 57b. A push rod 71 which is capable of protruding or receding from a distal end of the guide tube 70 is provided inside the guide tube 70, and a head portion 72 serving as an abutting member which abuts against the operation rod 65a in a manner capable of contacting or separating from the operation rod 65a is fixedly provided to a distal end of the push rod 71.

Furthermore, a distal end side of a driving wire 73 inserted in the guide tube 70 is coupled to the push rod 71, and a shape memory element 74 formed of a shape memory alloy is coupled to a proximal end side of the driving wire 73. Moreover, inside the guide tube 70, a push spring 75 is wound on an outer circumferential side of the driving wire 73 so as to bias the push rod 71 toward the front-side stopper 60 with a biasing force greater than the biasing force of the return spring 68.

For example, the shape memory element 74 is set to contract when heated and to expand when cooled, and is held inside the guide tube 70 in a state where expansion and contraction are allowed. Also, a heat source such as a Peltier device, not shown, is provided to the shape memory element 74, and the heat source is capable of heating or cooling the shape memory element 74 according to an operation state of the operation lever 24.

When cooled and expanded, the shape memory element 74 operates the driving wire 73 in a direction of releasing the biasing force of the push spring 75 (that is, in the direction toward the distal end side along the photographing optical axis O). The distal end side of the push rod 71 thereby protrudes from the guide tube 70, and presses the operation rod 65a against the biasing force of the return spring 68. The operation rod 65a thus moves to a position where the operation rod 65a abuts against the front-side stopper 60. In accordance with the movement of the operation rod 65a, the moving lens frame 65 moves the moving lens 66 to an advanced position where a first focal length (first optical property) set in advance is realized (see Fig. 3).

On the other hand, when heated and contracted, the shape memory element 74 operates the driving wire 73 in a direction against the biasing force of the push spring 75 (that is, in the direction toward the proximal end side along the photographing optical axis O). The distal end side of the push rod 71 is thereby retracted into the guide tube 70. The operation rod 65a is thus biased by the return spring 68 and is moved to a position where the operation rod 65a abuts against the adjustment ring 59. In accordance with the movement of the operation rod 65a, the moving lens frame 65 moves the moving lens 66 to a retracted position where a second focal length (second optical property) set in advance is realized (see Fig. 4). At this time, the proximal end side of the moving lens frame 65 overlaps with the protruding portion 58c of the first lens 58a without interfering with the protruding portion 58c.

In the image pickup unit 30 configured in the above manner, the advanced position of the moving lens 66 for realizing the first focal length is finely adjusted by adjustment of relative positions of respective fixed frames and the like at the time of assembly of the observation optical system unit 32, for example. That is, at the time of assembly of the observation optical system unit 32, the front-group lens frame 55, the rear-group lens frame 57, and the guide frame 47 are positioned relative to one another while checking the optical property and are fixed by an adhesive or the like in a state where the operation rod 65a is abutted against the front-side stopper 60.

On the other hand, with respect to the retracted position of the moving lens 66 for realizing the second focal length, fine adjustment is performed by positioning and fixing the front-group lens frame 55, the rear-group lens frame 57, and the guide frame 47 and then adjusting the rotation position of the adjustment ring 59 on the rear-group lens frame 57. That is, at the time of adjusting the retracted position of the moving lens 66, adjustment is performed in a state where the operation rod 65a is abutted against the adjustment ring 59, by rotating the adjustment ring 59 while checking the optical property and changing an abutment position of the operation rod 65a and the adjustment ring 59. Then, the adjustment ring 59 is fixed on the outer circumferential surface of the rear-group lens frame 57 by an adhesive or the like in a state where the position of the moving lens frame 65 in the direction of the photographing optical axis O and on the rear-group lens frame 57 is adjusted to an appropriate position.

According to the embodiment as described above, the first lens 58a positioned closest to an object side, among the rear-group lenses 58, is configured by a stepped lens including the proximal portion 58b, the outer circumference of which is held by the rear-group lens frame 57, and the protruding portion 58c formed to have an outer circumference smaller than the outer circumference of the proximal portion 58b and protruding into the region At where the moving lens frame 65 can advance or retract, and the moving lens frame 65 overlaps with the protruding portion 58c at the retracted position, and thus, an image may be picked up without reducing the resolution by preventing tilting of the moving lens frame 65 while not causing an increase in the size.

That is, by causing the proximal end side of the moving lens frame 65 to overlap with the protruding portion 58c of the first lens 58a, the region At where the moving lens frame 65 can advance or retract and a region occupied by the rear-group lenses 58 may be partially overlapped with each other in the direction of the photographing optical axis O. Accordingly, even in a case where the length in the direction of the photographing optical axis O is sufficiently secured so as to prevent tilting of the moving lens frame 65 and suppress a reduction in the resolution, an increase in the length of the observation optical system unit 32 in the direction of the photographing optical axis O may be appropriately prevented. Moreover, overlap between the moving lens frame 65 and the protruding portion 58c is realized by forming the protruding portion 58c by eliminating an unnecessary part, on the incident side of the first lens 58a, which does not optically function, and thus, the diameters of the moving lens frame 65, the guide frame 47, the rear-group lens frame 57 and the like do not have to be increased to realize the overlap, and an increase in the size in the outer diameter direction of the observation optical system unit 32 may be appropriately prevented.

Furthermore, according to the present embodiment adopting a configuration where the moving lens frame 65 slidingly contacts the rear-group lens frame 57 across the guide frame 47, tilting of the guide frame 47 with respect to the front-group lens frame 55 and the rear-group lens frame 57, which are fixed frames, has to be prevented to prevent tilting of the moving lens frame 65. Also in such a case, tilting of the guide frame 47 (the moving lens frame 65) may be prevented by causing a part of the proximal end side of the guide frame 47 to overlap with the protruding portion 58c of the first lens 58a, while not increasing the size of the observation optical system unit 32. Moreover, in the case where diameters of bonded lenses are approximately the same, an adhesive applied from an edge of one of the lenses penetrates into bonded surfaces, and the bonded surfaces may be damaged due to a stress caused by expansion/contraction at the time of hardening of the penetrating adhesive, thereby resulting in a negative effect on a passing light beam, that is, a reduction in the image quality, but because the protrusion 57d of the rear-group lens frame is separated from the protruding portion 58c of the lens, the adhesive fixing the rear-group lens frame 57 and the bonded lenses may be prevented from reaching the bonded surfaces by the action of surface tension.

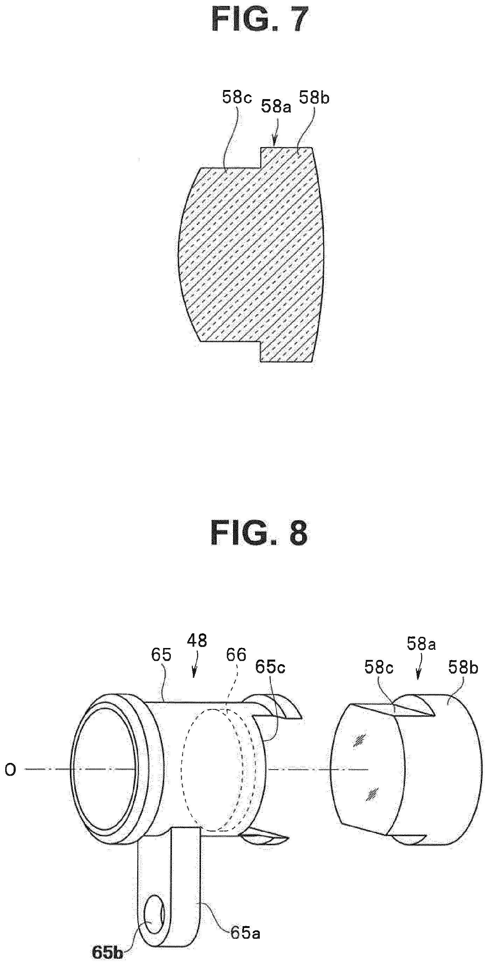

In the embodiment described above, an example is described where the first lens 58a is configured by bonding together, by a transparent adhesive or the like, the protruding portion 58c, which is a convex lens with a small diameter, and the proximal portion 58b, which is a concave lens with a large diameter, but the first lens 58a including the proximal portion 58b and the protruding portion 58c may be formed, for example, by cutting a part of an outer circumference of a single lens (lens raw material), as shown in Fig. 7.

Furthermore, the protruding portion 58c is not limited to be column-shaped, and, for example, in the case of picking up wide images of different aspect ratios, the protruding portion 58c may be formed by cutting only a part of the outer circumference, as shown in Fig. 8. In this case, cut-out portions 65c are provided to parts of the moving lens frame 65, on the proximal end side, in accordance with the shape of the protruding portion 58c so as to enable the moving lens frame 65 to overlap with the first lens 58a without causing an increase in the diameter of the moving lens frame 65 or the like.

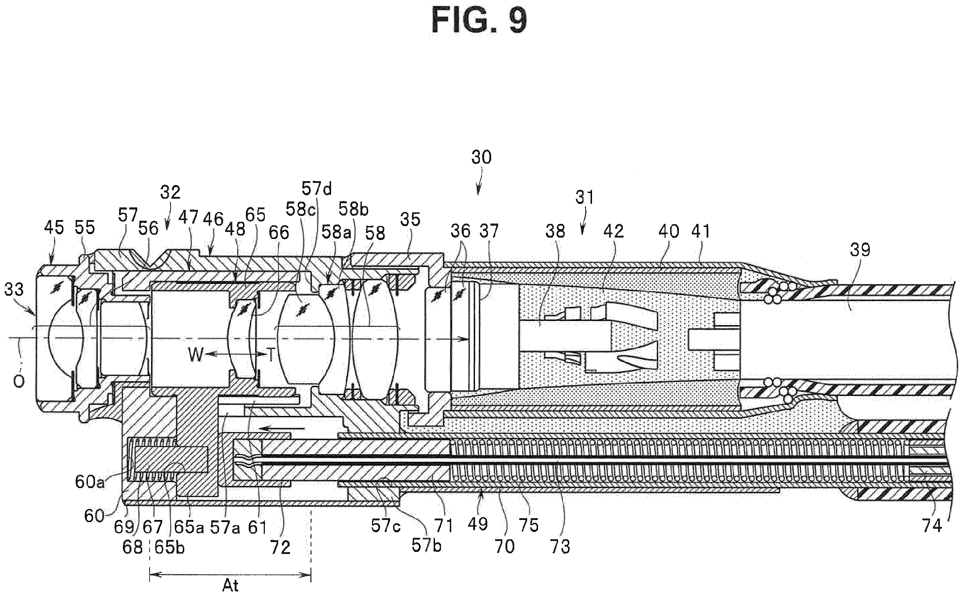

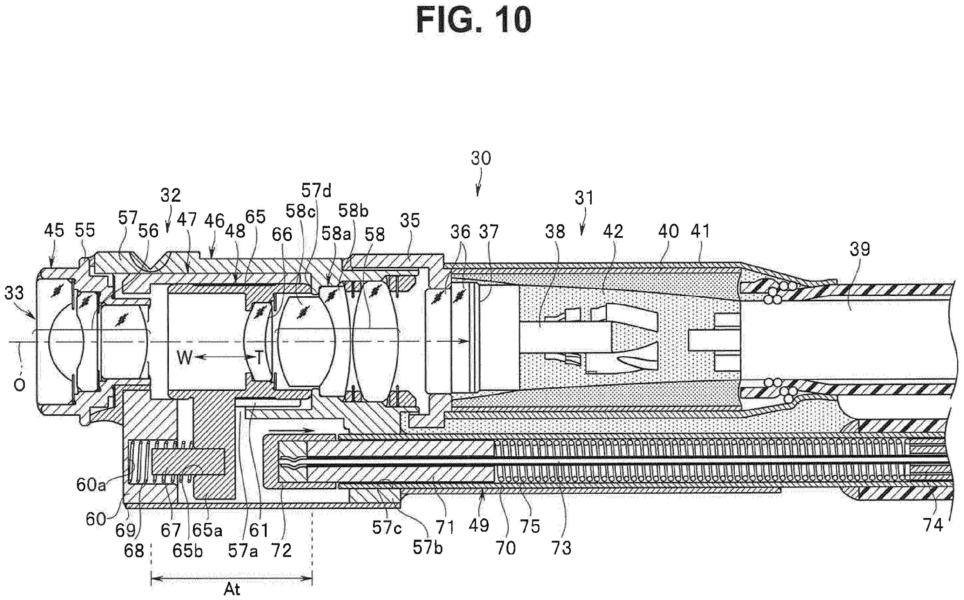

Moreover, for example, as shown in Figs. 9 and 10, in the case where a certain level of machining accuracy may be ensured for each member forming the rear-group lens unit 46, the moving lens unit 48 and the like, the retracted position of the moving lens frame 65 may be specified by optimizing the thickness of the abutting portion 57d provided to the rear-group lens frame 57 and causing the moving lens frame 65 to abut against a distal end surface of the abutting portion 57d. In the case where a shift from a planned position due to hardening of an adhesive is determined after fixed frames are fixed together, the optical property is corrected by adjusting the position of the guide frame.

According to-such a configuration, the moving lens frame 65 makes surface contact with the abutting portion 57d at the retracted position, and tilting of the moving lens frame 65 may be more appropriately prevented.

Note that the present invention is not limited to the embodiment described above, and various modifications and changes may be made within the technical scope of the present invention. For example, in the embodiment described above, an example of a configuration according to which the moving lens frame 65 is allowed to advance or retract inside the rear-group lens frame 57, which is a fixed frame, is described; however, the present invention is not limited to such an example, and a configuration may be adopted according to which the front-group lens frame 55, which is a fixed frame, is provided extending to the proximal end side, and the moving lens frame 65 is advanced or retracted inside the front-group lens frame 55, for example.

Moreover, in the embodiment described above, an example of a configuration according to which the moving lens frame 65 slidingly contacts the fixed frame (the rear-group lens frame 57) across the guide frame 47 is described; however, the present invention is not limited to such an example, and the guide frame 47 may be omitted and the moving lens frame 65 may be made to directly and slidingly contact the fixed frame (the front-group lens frame 55 or the rear-group lens frame 57).

It is needless to say that the configuration of the embodiment described above and the configuration of each modification may be combined as appropriate.

The present application claims priority from Japanese Patent Application No. 2015-172202 filed in Japan on September 1, 2015, the entire contents of which are incorporated in the present specification and claims by reference.

## Claims

1. An observation optical system unit comprising:
an observation optical system, of a focus switching type, including front-group lenses, a moving lens, and rear-group lenses in order from an object side;
a fixed frame holding the front-group lenses and the rear-group lenses; and
a moving frame holding the moving lens, slidingly contacting an inside of the fixed frame, and being capable of advancing and retracting along an optical axis direction of the observation optical system, wherein
a first lens positioned closest to the object side, among the rear-group lenses, is configured by a stepped lens including a proximal portion, an outer circumference of which is held by the fixed frame, and a protruding portion formed to have an outer circumference smaller than the outer circumference of the proximal portion and protruding into a region where the moving frame is allowed to advance and retract, and
the moving frame overlaps with the protruding portion at least at a position retracted to a side of the rear-group lenses along an optical axis of the observation optical system.

2. The observation optical system unit according to claim 1, comprising an abutting portion protruding in an inner diameter direction of the fixed frame, and specifying a retracted position of the moving frame by a surface on a distal end side in the optical axis direction abutting against the moving frame, wherein
the protruding portion of the first lens protrudes on the distal end side with respect to the abutting portion along the optical axis direction.

3. The observation optical system unit according to claim 2, wherein a step portion, of the first lens, formed by the proximal portion and the protruding portion is abutted against a surface on a proximal end side of the abutting portion in the optical axis direction.

4. The observation optical system unit according to claim 1, wherein the first lens is formed by bonding two lenses of different outer diameters.

5. The observation optical system unit according to claim 1, wherein the first lens is formed by cutting a part of an outer circumference of a single lens.

6. The observation optical system unit according to claim 1, comprising a guide frame interposed between the fixed frame and the moving frame, and guiding advancement and retraction, along the optical axis direction, of the moving frame slidingly contacting an inner circumferential surface, wherein
a proximal end side of the guide frame overlaps with the protruding portion of the first lens.

7. The observation optical system unit according to claim 6, wherein the guide frame includes a front-side stopper that specifies an advanced position of the moving frame by abutting against the moving frame.

8. An image pickup unit comprising:
the observation optical system unit according to claim 1; and
an image pickup device in which a subject image is formed by the observation optical system unit.

9. An endoscope comprising the image pickup unit according to claim 8 at a distal end portion of an insertion section inserted into a subject.
